(19) Europäisches Patentamt
European Patent Office
Office européen des brevets

(11) Publication number: **0 066 442**
**B1**

(12) # EUROPEAN PATENT SPECIFICATION

(45) Date of publication of patent specification: **31.07.85**

(51) Int. Cl.$^4$: **C 07 D 401/14,** C 07 D 213/65

(21) Application number: **82302664.6**

(22) Date of filing: **25.05.82**

(54) **Chemical process.**

(30) Priority: **27.05.81 GB 8116156**

(43) Date of publication of application:
**08.12.82 Bulletin 82/49**

(45) Publication of the grant of the patent:
**31.07.85 Bulletin 85/31**

(84) Designated Contracting States:
**AT BE CH DE FR GB IT LI LU NL SE**

(56) References cited:
**EP-A-0 004 793**
**EP-A-0 017 679**
**EP-A-0 049 173**
**FR-A-2 319 343**
**US-A-4 154 834**

**The file contains technical information submitted after the application was filed and not included in this specification**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Cooper, John**
**2 Waverly Drive**
**Sandown Park Tunbridge Wells Kent (GB)**
Inventor: **Mendelson, Wilford Lee**
**592 General Learned Road**
**King of Prussia Pennsylvania 19406 (US)**
Inventor: **White, George Raymond**
**16 Ashley Gardens**
**Harpenden Hertfordshire (GB)**
Inventor: **Levinson, Sidney Harry**
**2508 Stonybrook Lane**
**Drexel Hill Pennsylvania 19026 (US)**

(74) Representative: **Waters, David Martin, Dr. et al**
**Smith Kline & French Laboratories Ltd. Patent Department Mundells**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 066 442 B1

**Specification**

This invention provides a process for preparing 2-[4-(3-methoxy-2-pyridyl)butylamino]-5-(6-methyl-3-pyridylmethyl)-4-pyrimidone (III) or its acid addition salts. This compound is disclosed in United States Patent No. 4,154,834 issued May 15, 1979 and is known to be a useful pharmaceutical compound whose biological activity comprises blocking both histamine $H_1$ and $H_2$ receptors.

The process of this invention is represented by the following reaction sequence:

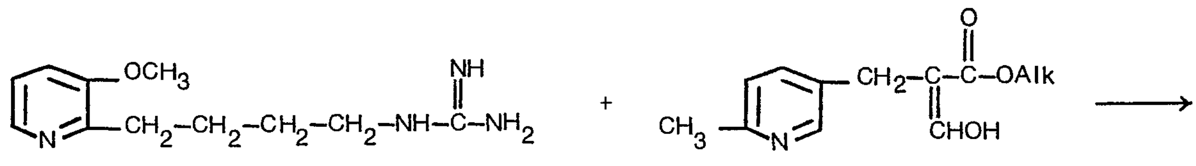

I II

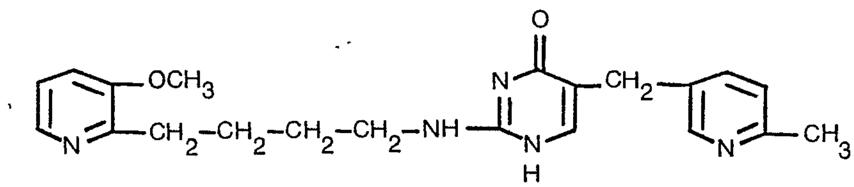

III

In this process, Alk is lower alkyl of 1-6 carbon atoms, usually methyl or ethyl.

Compound III is described in Example 46 of the above identified patent to have been prepared by the following reaction:

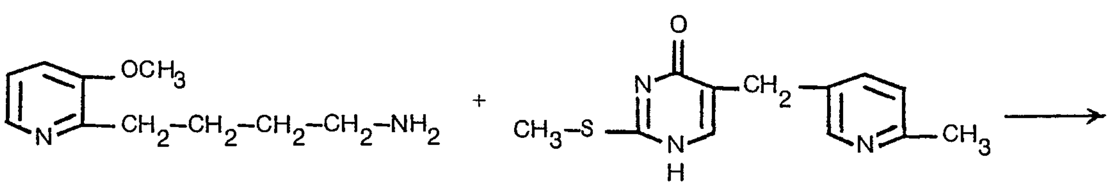

III

IV V

This prior art process presents a major environmental disadvantage in that unpleasant methyl mercaptan is produced as a by-product. Also the unit cost of chemical product is higher than that of the process of this invention due to a lower throughput, higher chemical cost of starting materials and greater number of steps starting from commercially available chemicals. Another prior art process involves the use of 2-nitroamino-5-(6-methyl-3-pyridylmethyl)-4-pyrimidone with the primary amine IV sketched above as detailed in Example 24 of EP-A-4793 and Example 1 of EP-A-17679. The present process avoids the use of nitroguanidine (which can explode when dry and detonated) and 2-nitroamino-1-pyrimidone derivatives.

The process of this invention comprises reacting N-[4-(3-methoxy-2-pyridyl)butyl]guanidine (I), isolated or generated in situ from one of its acid addition salts, with a molar equivalent or, preferably, an excess of a lower alkyl 2-formyl-3-(6-methyl-3-pyridyl)propionate (II) or its sodium or potassium salt in a solvent system in which the starting materials are substantially soluble. Such solvent systems are water, a halogenated hydrocarbon such as chloroform or methylene chloride, a lower alkanol such as propanol, isopropanol or butanol, an aromatic solvent such as benzene or toluene, an ether such as tetrahydrofuran, dimethylformamide, dimethylacetamide, dimethylsulfoxide, an ester solvent such as ethyl acetate or mixtures of such solvents such as water-halogenated hydrocarbon. The limited solubility of the guanidine reactant in salt form in organic solvents is a factor in the choice of solvent systems. When a biphasic solvent system such as water/chloroform is used a phase transfer catalyst may be useful such as a quaternary ammonium or phosphonium salt such as tricaprylylmethyl ammonium chloride, tetrabutylphosphonium halide or methyltrioctyl ammonium halide or a crown ether. The reaction is carried out under neutral or basic pH's such as about 9-10 maintaining the pH conditions by addition of alkali such as sodium or potassium hydroxide, sodium or potassium carbonate and sodium or potassium lower alkoxide.

The reaction is run at from ambient temperature up to reflux temperature of the reaction mixture until the condensation reaction is substantially complete. Reflux temperatures from 4—24 hours are convenient. The progress of the reaction may be monitored by thin layer chromatography or high pressure liquid

chromatography. The guanidine reactant may be optionally generated from its purified acid addition salt form in situ or used in the mother liquor of its formation in a one-pot reaction.

The pyrimidone (III) is produced in from 40—65% yields of good quality product. Only small amounts of the undesired isomers are formed during the cyclization reaction (5—10%). The end product is isolated and purified by methods generally known to the art. Purification is readily accomplished by chromatography over commercial silica or cellulose columns. Alternatively, it has been found that 2-[4-(3-methoxy-2-pyridyl)butylamino]-5-(6-methyl-3-pyridylmethyl)-4-pyrimidone as the base forms a crystalline trihydrate which is useful for purification of the end product by recrystallization from aqueous organic solvents which are not themselves hydrophilic.

The acid addition salts of the product (III) are easily prepared by reaction of the base with at least a stoichiometric quantity of the desired acid in an organic solvent such as isopropanol.

The ester starting materials (II) are compounds known to the art. The guanidine starting material (I) has not been reported previously. It, together with its acid addition salts, are part of this invention since these compounds not only have a unique use as intermediates but also have histamine $H_2$ blocking activity.

The following examples illustrate the process of this invention. Temperatures are Centrigrade.

Example 1

(A) 4-(3-methoxy-2-pyridyl)butylamine (2.0 g, 0.011 m) was added to 15 ml of water at 20°. The mixture was adjusted to pH 8.3 with 6N sulfuric acid. After adding 0.47 g of cyanamide, the mixture was heated to reflux. After 10 minutes, another 0.47 g of cyanamide was added while maintaining the pH at 8—8.5 and monitoring the progress of the reaction with high pressure liquid chromatography (60 methanol, 40 water, 1 acetic acid). After 5 hours at reflux 0.94 g of cyanamide was added, the pH was adjusted to 8.5 and the mixture was heated at reflux overnight. Reaction was determined to be complete. The volatiles were removed and the residue triturated with nitromethane to give 3.1 g of N-[4-(3-methoxy-2-pyridyl)butyl]guanidine as the hemisulfate, 75% pure with most of the impurity being dicyandiamide, mass spectrum shows m/e 222 with an appropriate fragmentation pattern.

(B) A mixture of 2.0 g (0.011 m) of 4-(3-methoxy-2-pyridyl)butylamine, 2.54 g (0.012 m) of S-methyl-pseudothiourea hydroidide and 10 ml of isopropanol was heated at reflux for 17 hours. The mixture was concentrated *in vacuo* to a syrup which solidified after trituration with nitro-methane to give 1.48 g (39%) of crude N-[4-(3-methoxy-2-pyridyl)butyl]guanidine hydriodide. Recrystallization from nitromethane gave a sample for analysis, m.p. 112°.

Anal. Calcd. for $C_{11}H_{18}N_4O\ H_2O{\cdot}HI$: C, 35.88; H, 5.75; N, 15.22. Found: C, 35.66, 35.69; H, 5.70, 5.77; N, 15.49, 15.51.

The base was regenerated and converted into the hemisulfate hydrate; m.p. 133—135°.

A mixture of 2.25 g (0.007 m) of N-[4-(3-methoxy-2-pyridyl)butyl]guanidine sulfate, 3.0 ml (0.015 m) of 25% sodium methoxide in methanol solution and 10 ml of isopropanol was heated to reflux. Ethyl 2-formyl-3-(6-methyl-3-pyridyl)propionate (2.67 g, 0.012 m) was added portionwise over one half hour. The progress of the reaction was monitored by high pressure liquid chromatography (HPLC). The pH was at 9.5—10 throughout. After refluxing overnight (17 hours), the mixture was concentrated to 3.26 g of dark oil (35% pure compared with an authentic sample by HPLC).

A 1.5 g portion of the oil was passed over a commercial activated magnesium silicate column (50 g, 17 × 3 cm) using 450 ml of 96:4 ethyl acetate/methanol and 250 ml of 92:8 ethyl acetate/methanol. The product containing fractions (determined using thin layer chromatography on silica gel plates using chloroform/methanol/ammonia) were combined, concentrated and treated with isopropanolic hydrogen chloride to give 2-[4-(3-methoxy-2-pyridyl)-butylamino]-5-(6-methyl-3-pyridylmethyl)-4-pyrimidone trihydrochloride, m.p. 205—207°, identical to an authentic sample in 98.2% purity by high pressure liquid chromatography. Mass spectrometry showed a molecular ion m/e 379.

Example 2

A. A mixture of 1.80 g (0.01 m) of 4-(3-methoxy-2-pyridyl) butylamine and 12 ml of water was stirred in an ice bath and adjusted to pH 8.1—8.2 with 6N hydrochloric acid then 0.48 g of cyanamide (98%) was added followed by heating to 60—65° again followed by another portion of 0.48 g of cyanamide. After adjusting the pH to 8—8.2, the mixture was heated at reflux for 17 hours.

The mixture was cooled and a mixture of 2.41 g of ethyl 2-formyl-3-(6-methyl-3-pyridyl)propionate and 15 ml of chloroform was added. A phase transfer agent (one drop of tricaprylylmethylammonium chloride) was also added. The pH was adjusted to 9—10. The mixture was heated at reflux with stirring for 16 hours. The cooled mixture was diluted with chloroform and water then analyzed by HPLC. The desired product was taken into chloroform which extract was dried and stripped of volatiles to give 3.60 g of oily product which was 50% pyrimidone. This material was purified over a magnesium silicate column as described above. The product containing fractions were combined and evaporated to give an oil which was treated with water and acetonitrile then stirred to give crystalline 2-[4-(3-methoxy-2-pyridyl)-butylamino]-5-(6-methyl-3-pyridylmethyl)-4-pyrimidonetrihydrate. A sample was recrystallized from 50% water-isopropanol, m.p. 55—60°.

Anal. Calcd. for $C_{21}H_{25}H_5O_2{\cdot}3H_2O$: C, 58.18; H, 7.21; N, 16.15. Found: C, 58.72, 58.63; H, 6.98, 6.96; N, 16.25, 16.14.

*The purified trihydrate was dissolved in isopropanol and reacted with isopropanolic hydrogen chloride* to give the trihydrochloride m.p. 202—204°.

B. The reaction above was repeated using 3.6 g (0.02 m) of amine with a reflux period, after adding the ester reactant, of 6 hours. The reaction mixture was worked up by separating the layers of the reaction mixture, washing the aqueous layer with chloroform, drying and stripping the combined organic layers *in vacuo* to give 13.5 g of pyrimidone product as an oil. This material was treated with water to give, after slurrying in nitromethane, 7.8 g of product as a trihydrate. Recrystallization from nitromethane and formation of the trihydrochloride salt gives pure product, m.p. 204—206°, identical with authentic 2-[4-(3-methoxy-2-pyridyl)butylamine]-5-(6-methyl-3-pyridylmethyl)-4-pyrimidone trihydrochloride.

Example 3

A mixture of 45 g (0.25 m) of 4-(3-methoxy-2-pyridyl)butylamine, about 1/4 of 45 ml of 50% aqueous cyanamide and 180 ml of n-propanol was adjusted to pH 7.5 with conc. sulfuric acid and heated at reflux briefly until a deep orange color was present. The remaining cyanamide was added as three portions interspersed with reflux periods. Total reflux time was 18 hours. Cooling separated a white precipitate which was separated, washed with propanol and oven-dried (50°) to give 55.9 g of N-[4-(3-methoxy-2-pyridyl)butyl]guanidine.

Sodium methoxide, 2.8 g (0.0525 m) in 11 ml of methanol, was added to a mixture of 11 g of the guanidine and 75 ml of n-propanol. After gentle heating, 2 g of ethyl 2-formyl-3-(6-methyl-3-pyridyl)propionate was added followed by a brief reflux period and then 9.1 g of ester in five portions. After a total of 10 hours reflux, 30 ml of volatiles was removed and 175 ml of water added. Cooling separated the product which separated first as an oil then as solid. The basic product was separated, washed with water and dried.

The base was dissolved in 150 ml of n-propanol and 10.5ml (3 m/e) of concentrated hydrochloric acid. After heating to reflux, 75 ml of solvent was distilled off. *n*-Propanol (50 ml) was added. Cooling separated 13.5 g (55.3%) of the desired trihydrochloride of Example 2 (98.9% pure by high pressure liquid chromatography).

**Claims**

1. The method of preparing 2-(pyridylbutylamino)pyrimidone of the formula;

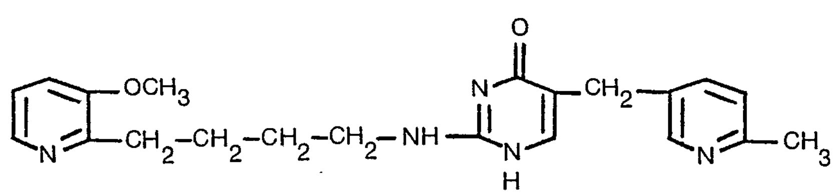

or one of its pharmaceutically acceptable salts, which comprises reacting a guanidine of the formula;

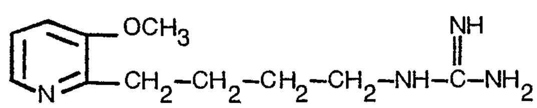

a 2-formyl-3-pyridinylpropionate of the formula:

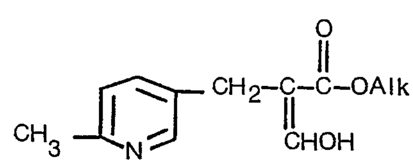

in which alk is lower alkyl of 1—6 carbon atoms, and, optionally, treating the basic product of the reaction with a pharmaceutically acceptable acid.

2. The method of claim 1 in which lower alkyl is methyl or ethyl and the reaction is carried out in a reaction medium which is neutral or weakly basic.

3. The method of claim 2 in which the solvent system is water/halogenated hydrocarbon.

4. The method of claim 3 in which a phase transfer agent is present.

5. The method of claims 1, 2, 3 or 4 in which the guanidine is generated in the reaction mixture from its acid addition salt.

6. N-[4-(3-Methoxy-2-pyridyl)butyl]guanidine and its acid addition salts.

7. N-[4-(3-Methoxy-2-pyridyl)butyl]guanidine as the base.

8. N-[4-(3-Methoxy-2-pyridyl)butyl]guanidine hemisulfate.

9. N-[4-(3-Methoxy-2-pyridyl)butyl]guanidine hydriodide.
10. 2-[4-(3-Methoxy-2-pyridyl)butylamino]-5-(6-methyl-3-pyridylmethyl)-4-pyrimidone trihydrate.

**Patentansprüche**

1. Verfahren zur Herstellung eines 2-(Pyridylbutylamino)pyrimidons der Formel

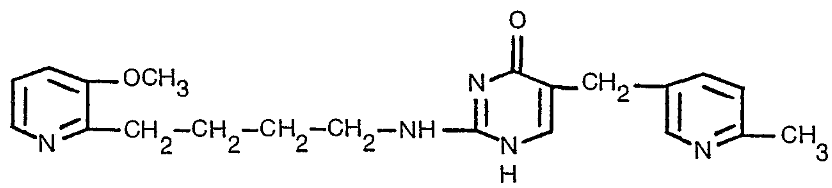

oder eines pharmazeutisch verträglichen Salzes, gekennzeichnet durch Umsetzen eines Guanidins der Formel

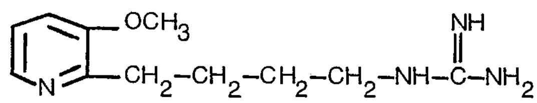

und eines 2-Formyl-3-pyridinylpropionsäureesters der Formel

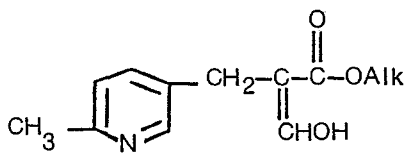

in der Alk einen Niederalkylrest mit 1 bis 6 Kohlenstoffatomen bedeutet, und gegebenenfalls Behandeln des basischen Reaktionsproduktes mit einer pharmazeutisch verträglichen Säure.

2. Verfahren nach Anspruch 1, wobei der Niederalkylrest eine Methyl- oder Äthylgruppe ist und die Umsetzung in einem Reaktionsmedium durchgeführt wird, das neutral oder schwach basisch ist.

3. Verfahren nach Anspruch 2, wobei das Lösungsmittelsystem Wasser/halogenierter Kohlenwasserstoff ist.

4. Verfahren nach Anspruch 3, wobei die Umsetzung in Gegenwart eines Phasentransfer-Mittels erfolgt.

5. Verfahren nach den Ansprüchen 1, 2, 3 oder 4, wobei das Guanidin im Reaktionsgemisch aus seinem Säureadditionssalz gebildet wird.

6. N-[4-(3-Methoxy-2-pyridyl)-butyl]-guanidin und seine Säureadditionssalze.

7. N-[4-(3-Methoxy-2-pyridyl)-butyl]-guanidin als Base.

8. N-[4-(3-Methoxy-2-pyridyl)-butyl]-guanidin-hemisulfat.

9. N-[4-(3-Methoxy-2-pyridyl)-butyl]-guanidin-hydrojodid.

10. 2-[4-(3-Methoxy-2-pyridyl)-butylamino]-5-(6-methyl-3-pyridylmethyl)-4-pyrimidon-trihydrat.

**Revendications**

1. La méthode de préparation d'un 2-(pyridylbutylamino)pyrimidone de la formule:

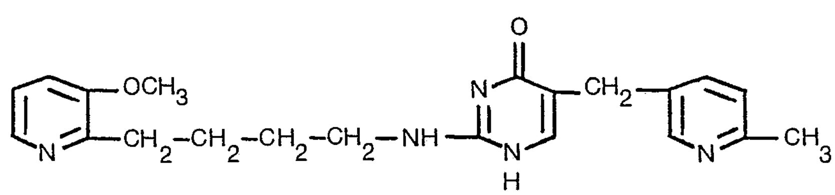

ou un de ses sels pharmaceutiquement acceptables, qui comprend la mise en réaction d'une guanidine de la formule:

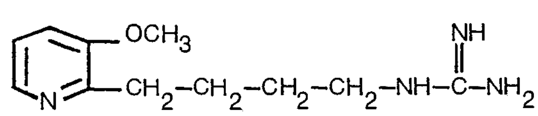

d'un 2-formyle-3-pyridinylepropionate de la formule:

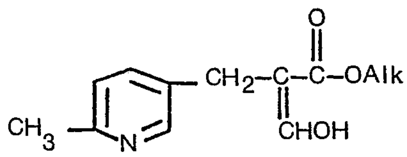

dans laquelle Alk est alcoyle plus faible ayant 1—6 atomes de carbone, et facultativement, en traitant le produit de base de la réaction avec une acide pharmaceutiquement acceptable.

2. La méthode de la revendication 1 dans laquelle l'alcoyle plus faible est du méthyle ou de l'éthyle et la réaction est réalisée dans un milieu de réaction qui est neutre ou faiblement basique.

3. La méthode de la revendication 2 dans laquelle le système de solvant est de l'eau/hydrocarbone halogénée.

4. La méthode de la revendication 3 dans laquelle un agent de transfert de phase est présent.

5. La méthode des revendications 1, 2, 3 ou 4 dans laquelle la guanidine est produite dans le mélange de réaction à partir de son sel d'addition d'acide.

6. N-[4-(3-Méthoxy-2-pyridyle)butyle]guanidine et ses sels d'addition d'acide.

7. N-[4-(3-Méthoxy-2-pyridyle)butyle]guanidine sous forme de base.

8. N-[4-(3-Méthoxy-2-pyridyle)butyle]guanidine hémisulfate.

9. N-[4-(3-Méthoxy-2-pyridyle)butyle]guanidine iodhydrate.

10. 2-[4-(3-Méthoxy-2-pyridyle(butylamino]-5-(6-méthyle-3-pyridylméthyle)-4-pyrimidone trihydrate.